# DEMANDE DE BREVET EUROPEEN

(11) **EP 1 842 425 A1**
(43) Date de publication de la demande: **10.10.2007**
(21) Numéro de dépôt: 07006362.3
(22) Date de dépôt: 28.03.2007
(51) Int. Cl.: A01N 37/02, A01N 37/06, A01P 17/00, A61K 8/36, A61K 31/201

(54) **Composition d'hygiène et/ou cosmétique et/ou désinfectante contentant au moins un agent anti-parasitaire répulsif et/ou insectifuge**

(30) Priorité: 31.03.2006 FR 0602817
(71) Demandeur: CID LINES N.V., 8900 Ieper (BE)
(72) Inventeur: Alasri, Richard, 8900 Ieper (BE); Goethals, Wouter, 8900 Ieper (BE)
(74) Mandataire: Duthoit, Michel Georges André

(57) **Abrégé**

La présente invention concerne une composition d'hygiène et/ou cosmétique et/ou désinfectante contenant au moins un agent antiparasitaire répulsif et/ou insectifuge.

Selon l'invention, ledit agent répulsif et/ou insectifuge est un acide gras.

La présente invention concerne également l'utilisation de ladite composition en tant que composition de traitement anti parasitaire et/ou répulsif et/ou insectifuge pour les arthropodes, notamment pour les insectes tels que les mouches, dans le traitement et/ou lavage des trayons et/ou de la mamelle des mammifères.

## Description

La présente invention concerne une composition d'hygiène et/ou cosmétique et/ou désinfectante contenant au moins un agent anti-parasitaire répulsif et/ou insectifuge.

Particulièrement, la présente invention concerne une composition d'hygiène et/ou cosmétique et/ou désinfectante en tant que composition anti parasitaire et/ou répulsive et/ou insectifuge pour les arthropodes, notamment les insectes tels que les mouches.

Plus particulièrement, la présente invention concerne une composition d'hygiène et/ou cosmétique et/ou désinfectante pour le traitement par trempage et/ou lavage et/ou pulvérisations des trayons et/ou de la mamelle des mammifères.

Il est connu depuis très longtemps l'utilisation de ce type de compositions pour tremper et/ou laver les trayons de la mamelle chez les mammifères. L'utilisation de telles compositions a pour but d'éviter et minimiser les mammites.

Les mammites sont des inflammations de la glande mammaire des mammifères, notamment des vaches laitières, plus ou moins aiguës dues à la pénétration de bactéries à l'intérieur de la mamelle. La mammite est une des maladies les plus répandues chez les vaches laitières et elle a pour conséquence de graves pertes économiques pour les éleveurs, notamment dues au fait que la vache mammiteuse produit moins de lait, qui sera, en outre, de moins bonne qualité et se vendra à un prix très bas.

La pénétration des bactéries dans la mamelle provoquant lesdites mammites, se produit le plus souvent à la fin de la traite lorsque le sphincter, orifice de sortie du lait, est encore ouvert.

Aujourd'hui, pour contrer cette pénétration de bactéries, la plupart des éleveurs trempe les trayons de chaque vache avec un produit filmant et désinfectant qui fera office de barrière aux micro organismes.

La plupart de ces produits de trempage connu contient, d'une part, une substance active désinfectante, qui permet de réduire la charge bactérienne au niveau des trayons, d'autre part, des émollients, pour adoucir la peau du trayon, évitant les gerçures et ainsi empêchant que le micro organisme ne rentre par ces crevasses, ainsi qu'un agent filmant, qui permet de faire tenir le produit sur le trayon et ainsi remonter le temps de contact entre le désinfectant et la surface à traiter.

Cela étant, beaucoup de facteurs peuvent être responsables de la présence de bactéries sur le trayon, comme par exemple une mauvaise hygiène des mains de la personne s'occupant de la traite.

Toutefois, l'inventeur a constaté que d'autres facteurs, sans rapport avec l'hygiène de l'exploitation, peuvent être responsables de l'apparition de mammites, par exemple les insectes et, en particulier les mouches qui vont, par leurs piqûres, propager les bactéries dans le troupeau.

Cependant, il s'avère que les compositions utilisées aujourd'hui pour empêcher les mammites ne sont satisfaisantes, car elles n'empêchent pas la transmission de l'infection par les insectes.

Par ailleurs, le traitement à base d'antibiotiques présente également des inconvénients, car ce traitement n'est pas toujours très efficace, les bactéries pouvant développer une résistance à leur égard.

En outre, les consommateurs sont souvent très inquiets quand il s'agit de la présence d'antibiotiques dans les produits animals.

Le but de la présente invention est de proposer une composition d'hygiène et/ou cosmétique, et/ou désinfectante contenant au moins un agent anti-parasitaire répulsif et/ou insectifuge qui pallie les inconvénients précités, notamment en ce qui concerne la transmission de l'infection par les insectes.

Un autre but de la présente invention est de proposer une composition d'hygiène et/ou cosmétique et/ou désinfectante destinée aux mammifères qui prévient l'apparition de mammites.

Un autre but de la présente invention est de proposer une composition d'hygiène et/ou cosmétique et/ou désinfectante destiné au corps humain et, notamment, pour les cheveux.

D'autres buts et avantages de la présente invention apparaîtront au cours de la description qui va suivre qui n'est donnée qu'à titre indicatif et qui n'a pas pour but de la limiter.

La présente invention concerne une composition d'hygiène et/ou cosmétique et/ou désinfectante contenant au moins un agent anti-parasitaire répulsif et/ou insectifuge.

Selon l'invention, ledit agent répulsif et/ou insectifuge est un acide gras.

Un des avantages de la composition telle que décrite dans la présente demande réside dans le fait qu'elle combine de façon synergique des substances d'hygiène et/ou cosmétique et/ou désinfectante et un agent anti-parasitaire répulsif et/ou insectifuge.

Les acides gras sont des acides carboxyliques présentant une longue chaîne hydrophobe pouvant être saturée ou insaturée.

Selon un mode particulier de la présente invention, l'acide gras est l'acide oléique. L'acide oléique est un des plus abondants des acides gras à chaîne longue, et se présente sous la forme d'un liquide (huile) qui ne se solidifie qu'à 4 °C.

Selon un mode particulier de la présente invention, ladite composition contient en outre des émollients et/ou un agent filmant.

Tels que déjà évoqués précédemment, les émollients permettent d'adoucir la peau du trayon, évitant les gerçures, et ainsi évitant que les micro-organismes ne rentrent pas dans ces crevasses.

En outre, l'agent filmant permet de faire tenir ladite composition sur le trayon et ainsi augmenter le temps de contact entre les divers constituants de la composition et la surface à traiter.

A titre d'exemple non limitatif, ladite composition selon la présente invention peut comprendre, entre autres :
- lode : 0,5 %
- Emollients (glycerol et/ou sorbitol) : 5 %
- Acide oléique : 1 %.
Un autre exemple d'une composition, à titre non limitatif, peut comprendre, entre autres :
- Acide lactique : 3 %,
- Emollients (sorbitol et/ou glycérol) : 5 %,
- Xanthan gum : 0,5 %,
- Acide oléique : 1 %.

Une telle composition, telle que décrite dans la présente invention est utilisée en tant que composition anti parasitaire et/ou répulsive et/ou insectifuge pour les arthropodes, notamment les insectes tels que les mouches.

Plus particulièrement, une telle composition, telle que décrite dans la présente invention est utilisée en tant que composition de traitement et/ou de lavage des trayons et/ou de la mamelle des mammifères.

En particulier, ladite composition est utilisée dans le traitement par trempage et/ou par lavage et/ou par pulvérisation et/ou par mousse des trayons des mammifères.

A ce sujet, le traitement et/ou le lavage des trayons des mammifères avant la traite est très important afin d'éviter l'infection des mamelles et/ou trayons par certaines bactéries. En effet, le nombre de micro organismes s'accroît près de l'orifice, ou sphincter, des trayons.

C'est à ce niveau que l'hygiène et les procédures de traite ont un rôle important à jouer pour éviter que ces microbes pénètrent la zone. Cette entrée peut être forcée par la trayeuse mécanique, surtout en fin de traite. Les trayons endommagés ou trop ouverts sont être plus facilement envahis.

Selon la présente invention, le mammifère peut être tout animal de traite, tel que tout mammifère laitier, tel que, par exemple, la vache, la brebis ou la chèvre, mais l'invention peut s'appliquer aux autres animaux confrontés aux mêmes problèmes.

Naturellement, d'autres modes de réalisation de la présente invention, à la portée de l'homme de l'art, auraient pu être envisagés sans pour autant sortir du cadre de l'invention.

## Revendications

1. Composition d'hygiène et/ou cosmétique et/ou désinfectante, contenant au moins un acide gras pour son utilisation en tant que composition répulsive et/ou insectifuge pour les arthropodes, notamment les insectes tels que les mouches, dans le traitement et/ou lavage des trayons et/ou de la mamelle des mammifères.

2. Composition selon la revendication 1, **caractérisée en ce que** l'acide gras est l'acide oléique.

3. Composition, selon les revendications 1 ou 2, **caractérisée en ce que** l'on effectue le traitement des trayons des mammifères par trempage et/ou par lavage et/ou pulvérisation et/ou par mousse.

4. Composition, selon la revendication 3, **caractérisée en ce que** le mammifère est la vache.

5. Composition, selon la revendication 3, **caractérisée en ce que** le mammifère est la brebis.

6. Composition, selon la revendication 3, **caractérisée en ce que** le mammifère est la chèvre.
